# EUROPÄISCHE PATENTSCHRIFT

(11) **EP 3 421 076 B1**
(45) Veröffentlichungstag und Bekanntmachung des Hinweises auf die Patenterteilung: **27.01.2021**
(21) Anmeldenummer: 18178712.8
(22) Anmeldetag: 20.06.2018
(51) Int. Cl.: A61M 39/10, A61M 39/16, A61M 39/22

(54) **MEDIZINISCHES VENTILSYSTEM**
MEDICAL VALVE SYSTEM
SYSTÈME DE VANNES MÉDICAL

(30) Priorität: 27.06.2017 DE 102017210794
(43) Veröffentlichungstag der Anmeldung: 02.01.2019
(73) Patentinhaber: B. Braun Melsungen AG, 34212 Melsungen (DE)
(72) Erfinder: BIERI, Sebastian, 6170 Schüpfheim (CH); DENK, Matthias, 3012 Bern (CH); DUDHANE, Mayur Suresh, Hyderabad 500034 (IN); EISEN, Frank, 6182 Escholzmatt (CH); KOBEL, Fritz, 3325 Hettiswil (CH); ZWYGART, Fritz, 3415 Hasle bei Burgdorf (CH)
(74) Vertreter: Patentanwälte Ruff, Wilhelm, Beier, Dauster & Partner mbB

(56) Entgegenhaltungen:
- WO-A1-2016/037646
- WO-A1-2016/037648
- US-A- 4 758 235
- US-A1- 2001 013 370
- US-A1- 2002 017 328

## Beschreibung

Die Erfindung betrifft ein Medizinisches Ventilsystem zur Verabreichung von wenigstens zwei Medikamentenflüssigkeiten mit
- einem Ventilgehäuse mit wenigstens zwei Medikamenteneinlässen zur Aufnahme jeweils einer der Medikamentenflüssigkeiten, die fluidleitend mit jeweils einem auf einer inneren Gehäuseumfangsfläche angeordneten Medikamentenauslass verbunden sind,
- einem zumindest abschnittsweise in dem Ventilgehäuse angeordneten Ventilkörper, der um eine Rotationachse relativ rotierbar zu der inneren Gehäuseumfangsfläche ist, mit einer äußerem Körperumfangsfläche und einer inneren Körperfläche und einem auf der äußeren Körperumfangsfläche angeordneten Durchlass,
- einem Spüleinlass zur Aufnahme einer Spülflüssigkeit, der fluidleitend mit einem Spülauslass verbunden ist und
- einem Ventilauslass zur Abgabe der Spülflüssigkeit und/oder wenigstens einer der Medikamentenflüssigkeiten, der fluidleitend mit dem Durchlass verbunden ist,
- wobei der Ventilkörper rotierbar ist in
   - wenigstens zwei Medikamentenstellungen, in denen der Durchlass jeweils derart fluidleitend mit einem der Medikamentenauslässe verbunden ist, dass die jeweilige Medikamentenflüssigkeit durch den Ventilauslass leitbar ist, und
   - wenigstens eine Spülstellung, in der der Durchlass über einen Spülkanal derart fluidleitend mit dem Spülauslass verbunden ist, dass die Spülflüssigkeit durch den Ventilauslass leitbar und derart Rückstände der ersten und/oder zweiten Medikamentenflüssigkeit aus dem Durchlass und dem Ventilauslass spülbar sind.

Ein derartiges medizinisches Ventilsystem ist aus der WO 2016/037648 A1 bekannt und zur seriellen Abgabe von Medikamenten bei der Infusionstherapie vorgesehen. Bei dem bekannten Ventilsystem ist der Spüleinlass auf einer Außenfläche des Ventilgehäuses angeordnet. Der Spülauslass ist auf der inneren Gehäuseumfangsfläche vorgesehen und fluidleitend mit dem Spüleinlass verbunden. In der Spülstellung durchströmt die Spülflüssigkeit daher zunächst das Ventilgehäuse radial von außen nach innen gerichtet, tritt sodann über eine erste Dichtstelle entweder direkt oder über den Spülkanal in den Durchlass an dem Ventilkörper ein, durchströmt diesen radial von außen nach innen und axial von oben nach unten gerichtet und tritt letztlich über eine zweite Dichtstelle in einen an dem Ventilgehäuse angeordneten Ventilauslass ein und wird durch diesen aus dem Ventilsystem abgegeben.

Die US 2002/0017328 A1 zeigt ein weiteres medizinisches Ventilsystem mit einem Ventilgehäuse und einem in dem Ventilgehäuse angeordneten Ventilkörper. Der Ventilkörper ist zur axialen Verbindung mit einer Spritze vorgesehen und weist zu diesem Zweck einen axialen Port auf. Der axiale Port ist fluidleitend mit einem radialen Port des Ventilkörpers verbunden. Je nach Drehstellung des Ventilkörpers in dem Ventilgehäuse ist der radiale Port insbesondere einem an dem Ventilgehäuse angeordneten Ventilauslass zustellbar.

Aus der WO 2016/037646 A1 ist ein weiteres medizinisches Ventilsystem mit einem Ventilgehäuse und einem Ventilkörper bekannt. Das Ventilgehäuse weist einen als saline inlet bezeichneten Spüleinlass und einen je nach Stellung des Ventilkörpers fluidleitend mit dem Spüleinlass verbindbaren Ventilauslass auf.

Die US 4 758 235 A zeigt ein weiteres medizinisches Ventilsystem mit einem Ventilgehäuse und einem in dem Ventilgehäuse zwischen unterschiedlichen Stellungen rotierbaren Ventilkörper. An dem Ventilgehäuse sind mehrere Ventileinlässe und ein Ventilauslass angeordnet, wobei jeweils einer der Ventileinlässe in Abhängigkeit der Stellung des Ventilkörpers fluidleitend mit dem Ventilauslass verbindbar ist.

Die US 2001/0013370 A1 zeigt ein medizinisches Vier-Wege-Ventil mit einem Ventilgehäuse und einem darin drehbeweglich gelagerten Ventilkörper. Das Ventilgehäuse weist mehrere axial abragende Ports auf, wobei einer der Ports als Ventilauslass fungiert. Der Ventilkörper weist einen axialen Port auf, der je nach Stellung des Ventilkörpers insbesondere fluidleitend mit dem Ventilauslass des Ventilgehäuses verbindbar ist.

Aufgabe der Erfindung ist es, ein medizinisches Ventilsystem der eingangs genannten Art zu schaffen, dass eine verbesserte Dichtigkeit bei gleichzeitig optimierter Bauraumausnutzung ermöglicht.

Diese Aufgabe wird durch ein medizinisches Ventilsystem mit den Merkmalen des Anspruchs 1 gelöst. Infolge der erfindungsgemäßen Lösung steht an dem Ventilgehäuse mehr Bauraum zur Anordnung der Medikamenteneinlässe zur Verfügung. Demzufolge wird eine optimierte Bauraumausnutzung erreicht. Auf diese Weise können zudem potentiell undichte Dichtstellen zwischen dem Ventilkörper und dem Ventilgehäuse eingespart und folglich ein Risiko einer Undichtigkeit reduziert werden. Unter einem Ventilauslass im Sinne der Erfindung, ist der in der üblichen Durchströmungsrichtung des Ventilsystems letzte Abschnitt der fluidleitenden Geometrie des Ventilsystems zu verstehen. Insoweit ist der Ventilauslass als ein Abschnitt der fluidleitenden Geometrie des Ventilsystems zu verstehen, durch welchen Spül- oder Medikamentenflüssigkeit das Ventilsystem verlässt. Vorteilhafterweise ist der Ventilauslass durch eine in den Ventilkörper eingebrachte Durchgangsöffnung, insbesondere eine Bohrung, gebildet. Der Ventilauslass kann alternativ oder zusätzlich eine an dem Ventilkörper angeordnete Hülse, einen Port oder dergleichen umfassen. Unter einem Spüleinlass im Sinne der Erfindung ist ein Abschnitt der fluidleitenden Geometrie des Ventilkörpers zu verstehen, durch welchen Spülflüssigkeit in der Spülstellung ohne vorheriges Durchströmen des Ventilgehäuses leitbar ist. Insoweit ist unter dem Spüleinlass eine in den Ventilkörper eingebrachte Durchgangsöffnung, insbesondere eine Bohrung, zu verstehen. Unter einer Medikamentenflüssigkeit im Sinne der Erfindung sind nicht ausschließlich solche flüssigen Zusammensetzungen zu verstehen, die medizinisch wirksam sind. Vielmehr ist der Begriff Medikamentenflüssigkeit weit auszulegen und umfasst demnach auch Blutpräparate, Nährlösungen, Kontrastmittel oder dergleichen. Im Sinne der Erfindung umfasst der Begriff Spülflüssigkeit solche medizinisch neutralen Flüssigkeiten, die zu einem Durchspülen des Ventilsystems und/oder einer Verabreichung an einen Patienten geeignet sind. Die Spülflüssigkeit kann bevorzugt eine Kochsalzlösung sein. In dieser Beschreibung verwendete Positions- und/oder Richtungsangaben, wie oben, unten, vorne, hinten, innen, außen oder dergleichen, beziehen sich auf eine gedachte Orientierung des Ventilsystems im Raum, bei der die Rotationsachse des Ventilkörpers im Wesentlichen fluchtend mit der Raumhochachse und derart ausgerichtet ist, dass der Ventilauslass in Bezug auf die Raumhochachse unterhalb der Medikamenteneinlässe angeordnet ist.

In Ausgestaltung der Erfindung ist der Spüleinlass auf der inneren Körperfläche des Ventilkörpers angeordnet ist und der Spülauslass auf der äußeren Körperumfangsfläche des Ventilkörpers angeordnet ist, so dass die Spülflüssigkeit in der Spülstellung von innen nach außen gerichtet durch den Ventilkörper leitbar ist. Vorteilhafterweise ist der Spüleinlass über eine im Wesentlichen radial zu der Rotationsachse erstreckte Durchgangsbohrung fluidleitend mit dem Spülauslass verbunden.

In weiterer Ausgestaltung der Erfindung weist der Ventilkörper einen Zuleitungskanal, der fluidleitend mit dem Spülauslass verbunden und im Wesentlichen axial zur Rotationsachse des Ventilkörpers erstreckt ist, sowie einen Ableitungskanal auf, der fluidleitend mit dem Durchlass und dem Ventilauslass verbunden und im Wesentlichen axial zu dem Zuleitungskanal erstreckt ist. Demzufolge wird eine fluchtende und vertikale Ausrichtung von einer mit dem Zuleitungs- und dem Ableitungskanal verbindbaren Schlauchzuleitung bzw. Schlauchableitung erreicht. Diese Ausgestaltung der Erfindung ermöglicht demnach eine strömungsoptimierte Anordnung dieser Schlauchleitungen und somit eine optimierte Durchströmung des Ventilsystems. Der Zuleitungskanal und der Ableitungskanal sind durch eine Trennwand derart fluiddicht voneinander getrennt, dass ein direkter Übertritt der Spülflüssigkeit von dem Zuleitungskanal in den Ableitungskanal - unter Umgehung des Spülauslasses, des Spülkanals und des Durchlasses - verhindert ist. Der Zuleitungskanal ist über den Spüleinlass fluidleitend mit dem Spülauslass verbunden.

In weiterer Ausgestaltung der Erfindung ist der Spülauslass unterhalb des Durchlasses angeordnet, wobei der Ventilkörper einen ersten Überleitungskanal aufweist, der den Spüleinlass fluidleitend mit dem Zuleitungskanal verbindet, sowie einen zweiten Überleitungskanal aufweist, der den Durchlass fluidleitend mit dem Ableitungskanal verbindet, und wobei die Überleitungskanäle in axialer Richtung des Ventilkörpers zumindest abschnittsweise nebeneinander angeordnet sind. Derart wird eine funktionsgerechte Fluidführung erreicht.

In weiterer Ausgestaltung der Erfindung sind der erste Überleitungskanal und der zweite Überleitungskanal wenigstens abschnittsweise im Wesentlichen zueinander parallel erstreckt und durch eine im Wesentlichen axial erstreckte Trennwand voneinander getrennt.

In weiterer Ausgestaltung der Erfindung ist der erste Überleitungskanal und/oder der zweite Überleitungskanal zu der Rotationsachse schräg erstreckt.

In weiterer Ausgestaltung der Erfindung ist der Spülkanal ringförmig zwischen der inneren Gehäuseumfangsfläche und der äußeren Körperumfangsfläche ausgebildet. Demzufolge kann eine nahezu vollständige Befüllung des Spülkanals mit der Spülflüssigkeit erreicht und ein Risiko von nicht befüllten Bereichen reduziert werden. Somit wird die Bildung unerwünschter und für einen Patienten potentiell gefährlicher Lufteinschlüsse in der fluidleitenden Geometrie des Ventilsystems vermieden und ein besonders anwendungssicheres Ventilsystem erreicht. Die Ringform des Spülkanals kann vorteilhafterweise durch eine entsprechende Anordnung des Spülauslasses und des Durchlasses erreicht werden, wobei insbesondere eine Art Strömungshindernis zwischen dem Spülauslass und dem Durchlass angeordnet sein kann.

In weiterer Ausgestaltung der Erfindung weist der Ventilkörper wenigstens eine radiale Abragung auf, die derart an der Körperumfangsfläche angeordnet ist, dass wenigstens ein Strömungshindernis in dem Spülkanal ausgebildet ist. Das Strömungshindernis ist vorteilhafterweise derart angeordnet, dass eine strömungstechnischer Kurzschluss zwischen dem Spülauslass und dem Durchlass vermieden wird. Die Abragung verschließt demnach den Querschnitt eines Spülkanalabschnittes zumindest teilweise. Durch diese Ausgestaltung der Erfindung wird eine verbesserte Befüllung des Spülkanals mit der Spülflüssigkeit und letztlich eine gesteigerte Anwendungssicherheit erreicht.

In weiterer Ausgestaltung der Erfindung ist eine erste Rastverbindung zwischen den Ventilgehäuse und dem Ventilkörper vorgesehen zum rotatorischen Festlegen des Ventilkörpers an dem Ventilgehäuse. Demzufolge wird durch die erste Rastverbindung eine definierte rotatorische Ausrichtung des Ventilkörpers relativ zu dem Ventilgehäuse, insbesondere in der Spülstellung und/oder der ersten sowie der zweiten Medikamentenstellung ermöglicht. Einem ungewollten Verlassen der Spülstellung und/oder der Medikamentenstellungen wird derart entgegengewirkt und eine verbesserte Anwendungssicherheit des Ventilsystems erreicht.

In weiterer Ausgestaltung der Erfindung ist eine zweite Rastverbindung zwischen dem Ventilgehäuse und dem Ventilkörper vorgesehen, die derart angeordnet ist, dass der Ventilkörper lediglich in eine Drehrichtung um die Rotationsachse rotierbar ist. Durch die zweite Rastverbindung kann demnach eine vordefinierte Abfolge von seriell einnehmbaren Stellungen des Ventilkörpers erreicht werden. Einem unbeabsichtigten Zurückdrehen des Ventilkörpers beispielsweise in eine bereits zuvor eingenommene Medikamentenstellung kann derart entgegengewirkt werden. Dies ist eine besonders sicher handhabbare Ausgestaltung des erfindungsgemäßen Ventilsystems.

In weiterer Ausgestaltung der Erfindung weist die erste und/oder zweite Rastverbindung jeweils eine/eine an einer Umfangsfläche des Ventilkörpers umlaufend angeordnete Profilierung auf. Die Profilierung kann radiale Abragungen und/oder radiale Vertiefungen aufweisen.

In weiterer Ausgestaltung der Erfindung weist der Ventilkörper wenigstens ein manuell betätigbares Entriegelungselement auf, das derart ausgestaltet ist, dass die Profilierung elastisch radial aufweitbar und derart die erste und/oder zweite Rastverbindung zwischen dem Ventilkörper und dem Ventilgehäuse lösbar ist. Demnach ist das Entriegelungselement zwischen einer Verriegelungsstellung, in der die erste und/oder zweite Rastverbindung unbeweglich verrastet sind/ist, und einer Entriegelungsstellung, in der die erste und/oder zweite Rastverbindung beweglich sind/ist, verlagerbar. Vorteilhafterweise weist der Ventilkörper zwei Entriegelungselemente auf, wobei ein Entriegelungselement der ersten Rastverbindung und das andere Entriegelungselement der zweiten Rastverbindung zugeordnet ist. Alternativ oder zusätzlich kann das Entriegelungselement an dem Ventilgehäuse angeordnet sein. Einem ungewollten Lösen der ersten und/oder zweiten Rastverbindung wird somit entgegengewirkt.

In weiterer Ausgestaltung der Erfindung ist eine erste Anschlusshülse mit einer Längsbohrung zur fluiddichten Aufnahme einer Spülflüssigkeitszuleitung vorgesehen, wobei die Längsbohrung fluidleitend mit dem Spüleinlass verbunden ist und wobei die erste Anschlusshülse derart stirnseitig und koaxial zu der Rotationsachse an dem Ventilkörper festgelegt ist, dass der Ventilkörper relativ rotierbar zu der ersten Anschlusshülse ist. Vorteilhafterweise kann die Spülflüssigkeitszuleitung eine Schlauchleitung sein. Durch die derart rotierbare erste Anschlusshülse wird einerseits einem ungewollten Verdrehen der Spülflüssigkeitszuleitung durch eine Rotation des Ventilkörpers und andererseits einem ungewollten Verdrehen des Ventilkörpers durch eine Rotation der Spülflüssigkeitszuleitung entgegengewirkt. Durch diese Ausgestaltung der Erfindung wird eine verbesserte Anwendungssicherheit des erfindungsgemäßen Ventilsystems erreicht.

In weiterer Ausgestaltung der Erfindung ist eine zweite Anschlusshülse mit einer Längsbohrung zur fluiddichten Aufnahme einer Ventilableitung vorgesehen, wobei die Längsbohrung fluidleitend mit dem Durchlass verbunden ist und die zweite Anschlusshülse derart stirnseitig und koaxial zu der Rotationsachse an dem Ventilkörper festgelegt ist, dass der Ventilkörper relativ rotierbar zu der zweiten Anschlusshülse ist. Vorteilhafterweise kann die Ventilableitung eine Schlauchleitung sein. Durch die derart rotierbare zweite Anschlusshülse wird einerseits einem ungewollten Verdrehen der Ventilableitung durch eine Rotation des Ventilkörpers und andererseits einem ungewollten Verdrehen des Ventilkörpers durch eine Rotation der Ventilableitung entgegengewirkt. Durch diese Ausgestaltung der Erfindung wird eine nochmals verbesserte Anwendungssicherheit des erfindungsgemäßen Ventilsystems erreicht.

In weiterer Ausgestaltung der Erfindung sind/ist die erste Anschlusshülse und/oder die zweite Anschlusshülse rotationsfest an dem Ventilgehäuse befestigt. Rotationsfest befestigt meint, dass eine Rotation zwischen der ersten und/oder der zweiten Anschlusshülse und dem Ventilgehäuse unterbunden ist. Demzufolge wird eine drehstarre Anbindung der mit der jeweiligen Anschlusshülse verbindbaren Spülflüssigkeitszuleitung bzw. Ventilableitung an das Ventilgehäuse erreicht. Einem ungewollten Verdrehen der Spülflüssigkeitszuleitung und/oder der Ventilableitung durch eine Rotation des Ventilkörpers zwischen den verschiedenen Stellungen wird somit entgegengewirkt.

In weiterer Ausgestaltung der Erfindung weisen/weist die erste und/oder die zweite Anschlusshülse jeweils eine/eine Abdeckplatte auf, die derart angeordnet sind/ist, dass jeweils ein/ein zwischen dem Ventilkörper und dem Ventilgehäuse ausgebildeter Radialspalt stirnseitig abgedeckt ist. Ein solcher Radialspalt kann für Reinigungszwecke schlecht zugänglich sein, so dass es zu einer unerwünschten und potentiell gesundheitsgefährdenden Keimansammlung kommen kann. Durch die derartige Ausgestaltung der Erfindung wird dem entgegengewirkt.

In weiterer Ausgestaltung der Erfindung weist der Ventilkörper einen Verbindungskanal auf, der in wenigstens einer der Medikamentenstellungen fluidleitend mit dem Spülkanal verbunden ist, und die zweite Anschlusshülse weist eine von der Außenumfangsfläche der Anschlusshülse in deren Längsbohrung reichende Querbohrung auf, wobei die zweite Anschlusshülse ausgehend von der jeweiligen Medikamentenstellung des Ventilkörpers relativ zu diesem rotierbar ist in eine Back-Primingstellung, in der die Querbohrung fluidleitend mit dem Verbindungskanal verbunden ist. Durch diese Ausgestaltung der Erfindung wird eine Rückspülung des jeweiligen Medikamenteneinlasses mit Spülflüssigkeit ermöglicht. Befindet sich der Ventilkörper in einer der Medikamentenstellungen und die zweite Anschlusshülse in der Primingstellung ist demzufolge eine fluidleitende Verbindung zwischen dem Spüleinlass, dem Spülauslass, dem Spülkanal, dem Verbindungskanal, der Querbohrung und der Längsbohrung der zweiten Anschlusshülse sowie dem Durchlass, dem jeweiligen Medikamentenauslass und dem korrespondierenden Medikamenteneinlass hergestellt. Entlang dieser fluidleitenden Verbindung kann demnach Spülflüssigkeit durch den Medikamenteneinlass ausgespült und dieser derart "geprimed" werden. Zu diesem Zweck ist vorteilhafterweise der Ventilauslass oder die Ventilableitung, beispielsweise mittels eines Stopfens oder einer Klemme oder dergleichen, verschlossen.

In weiterer Ausgestaltung der Erfindung ist ein zwischen der äußeren Körperumfangsfläche und der inneren Gehäuseumfangsfläche angeordnetes Dichtelement vorgesehen zum Abdichten eines Radialspaltes zwischen dem Ventilkörper und dem Ventilgehäuse. Das Dichtelement kann vorteilhafterweise gummielastische Materialeigenschafen und demnach eine elastomere Materialzusammensetzung aufweisen. Auf diese Weise kann, insbesondere im Vergleich zu einem bloßes Verpressen der äußeren Körperumfangsfläche mit der inneren Gehäuseumfangsfläche, eine besonders zuverlässige Abdichtung des Ventilsystems erreicht werden.

In weiterer Ausgestaltung der Erfindung ist eine stirnseitig an dem Ventilgehäuse angeordnete Abdeckvorrichtung vorgesehen mit einem Grundkörper mit einer durchgehenden Öffnung zur Aufnahme einer Schlauchleitung sowie mindestens einem im Bereich mindestens eines der Medikamenteneinlässe angeordneten Abdeckelement, dass derart gestaltet ist, dass es die manuelle Zugänglichkeit zu dem Medikamenteneinlass einschränkt. Die Abdeckvorrichtung kann vorteilhafterweise als separates und mit dem Ventilgehäuse verbindbares Bauelement ausgestaltet sein. Vorteilhafterweise ist das Abdeckelement als Spritzgussbauteil ausgeführt und weist eine Kunststoffmaterialzusammensetzung auf. Durch diese Ausgestaltung der Erfindung wird einem ungewollten Lösen von mit den Medikamenteneinlässen verbindbaren Medikamentenzuleitungen entgegengewirkt und somit ein besonders anwendungssicheres Ventilsystem geschaffen.

In weiterer Ausgestaltung der Erfindung weist das Abdeckelement ein Verbindungsmittel auf, das derart gestaltet ist, dass eine Medikamentenzuleitung an dem Abdeckelement formschlüssig arretierbar ist. Zu diesem Zweck kann das Abdeckelement vorteilhafterweise eine Klemm- oder Rasteinheit aufweisen, die mit einem Abschnitt der Medikamentenzuleitung lösbar verbindbar ist.

In weiterer Ausgestaltung der Erfindung ist das Abdeckelement mittels eines Befestigungsmittels relativ verschwenkbar zu dem Grundkörper an diesem befestigt, insbesondere mittels eines Filmscharniers. Vorteilhafterweise ist das Abdeckelement derart relativ verschwenkbar zwischen einer Arretierstellung, in der das Abdeckelement dem Abschnitt der Medikamentenzuleitung befestigt ist, und einer Freigabestellung, in der keine solche Befestigung vorliegt.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer, perspektivischer Darstellung eine erste Ausführungsform eines erfindungsgemäßen Ventilsystems,
- Fig. 2: in schematischer, perspektivischer Darstellung das Ventilgehäuse des Ventilsystems nach Fig. 1 aus einer oberen Perspektive,
- Fig. 3: das Ventilgehäuse nach Fig. 2 aus einer unteren Perspektive,
- Fig. 4: in schematischer, perspektivischer Schnittdarstellung das Ventilgehäuse nach Fig. 2 und 3,
- Fig. 5: in schematischer, perspektivischer Darstellung den Ventilkörper des Ventilsystems nach Fig. 1,
- Fig. 6 u. 7: in schematischer, perspektivischer Schnittdarstellung den Ventilkörper nach Fig. 5 in einer ersten Schnittebene (Fig. 6) und in einer zu dieser um etwa 90° gedrehten Schnittebene (Fig. 7),
- Fig. 8 u. 9: in schematischer, perspektivischer Schnittdarstellung das Ventilsystem nach Fig. 1 entlang einer ersten Schnittebene (Fig. 8) und einer zu dieser um etwa 90° gedrehten Schnittebene (Fig. 9),
- Fig. 10: in schematischer, perspektivischer Darstellung einen ersten Rastbereich des Ventilsystems nach Fig. 1,8 sowie 9,
- Fig. 11: in schematischer, perspektivischer Darstellung einen zweiten Rastbereich des Ventilsystems nach Fig. 1,8 sowie 9,
- Fig. 12: in schematischer Seitenansicht eine zweite Ausführungsform eines erfindungsgemäßen Ventilsystems,
- Fig. 13 u. 14: in schematischer Längsschnittdarstellung das Ventilsystem nach Fig. 12 in einer Medikamentenstellung (Fig. 13) und in einer Spülstellung (Fig.14),
- Fig. 15: in schematischer Seitenansicht eine dritte Ausführungsform eines erfindungsgemäßen Ventilsystems,
- Fig. 16: in schematischer Längsschnittdarstellung das Ventilsystem nach Fig. 15 in einer Medikamentenstellung,
- Fig. 17: in schematischer, perspektivischer Darstellung eine vierte Ausführungsform eines erfindungsgemäßen Ventilsystems,
- Fig. 18: in schematischer Seitenansicht das Ventilsystem nach Fig. 17,
- Fig. 19: in schematischer Querschnittsdarstellung das Ventilsystem nach Fig. 17 und 18 in einer Schnittebene F-F,
- Fig. 20: in schematischer Längsschnittdarstellung das Ventilsystem nach Fig. 17 bis 19 in einer Schnittebene G-G,
- Fig. 21: in schematischer Längsschnittdarstellung das Ventilsystem nach Fig. 17 bis 20 in einer Schnittebene E-E,
- Fig. 22: in schematischer Draufsicht eine fünfte Ausführungsform eines erfindungsgemäßen Ventilsystems,
- Fig. 23: in schematischer Längsschnittdarstellung das Ventilsystem nach Fig. 22 in einer Schnittebene T-T,
- Fig. 24: in schematischer, perspektivischer Darstellung eine Abdeckvorrichtung eines erfindungsgemäßen Ventilsystems,
- Fig. 25: in schematischer, perspektivischer Darstellung eine sechste Ausführungsform eines erfindungsgemäßen Ventilsystems aufweisend die Abdeckvorrichtung nach Fig. 24 und
- Fig. 26: in schematischer Längsschnittdarstellung eine sechste Ausführungsform eines erfindungsgemäßen Ventilsystems aufweisend ein Dichtelement.

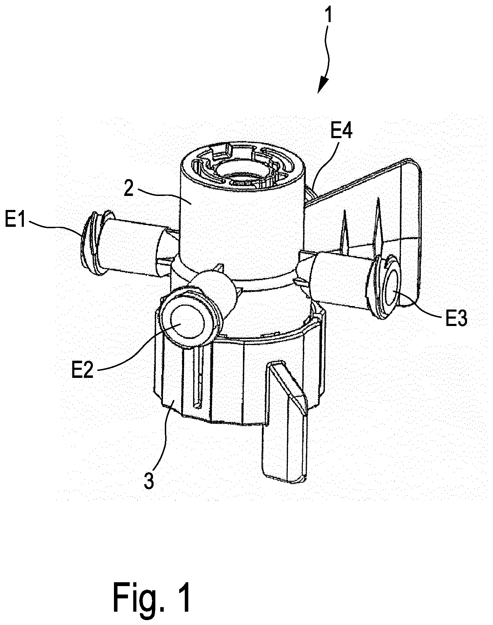

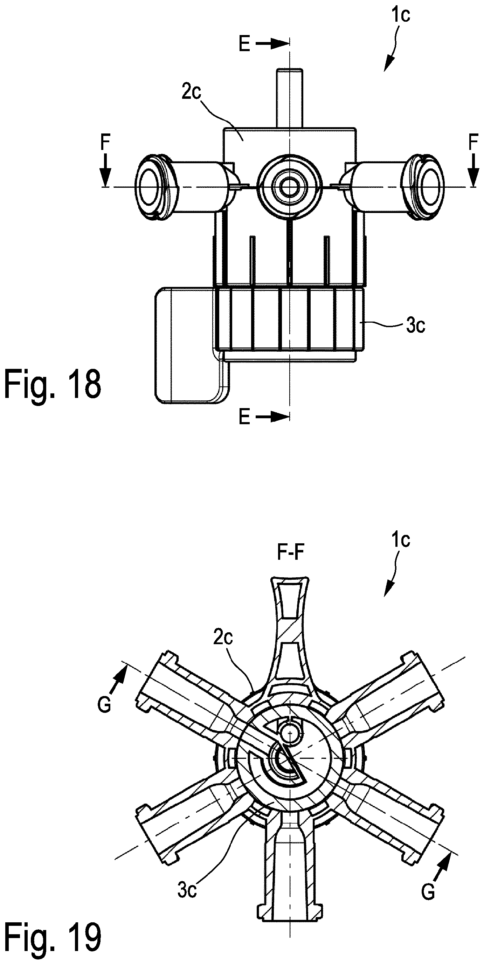

Anhand der Fig. 1 bis 26 sind verschiedene erfindungsgemäße medizinische Ventilsysteme 1 bis 1f dargestellt, die insbesondere für eine Verwendung bei der Infusionstherapie vorgesehen sind. Besonders vorteilhaft sind die Ventilsysteme 1 bis 1f für die Verabreichung von Zytostatika.

Jedes der Ventilsysteme 1 bis 1f weist ein Ventilgehäuse 2 bis 2f und einen Ventilkörper 3 bis 3f auf. Der strukturelle sowie funktionelle Aufbau der Ventilgehäuse 2 bis 2f und der Ventilkörper 3 bis 3f ist bei den verschiedenen Ventilsystemen 1 bis 1f weitestgehend identisch. Die im Wesentlichen identischen Merkmale der Ventilgehäuse 2 bis 2f und der Ventilkörper 3 bis 3f werden daher im Folgenden näher anhand des Ventilgehäuses 2 und des Ventilkörpers 3 des Ventilsystems 1 nach den Fig. 1 bis 11 erläutert. Diese Merkmale sind für den Fachmann ohne weiteres auf die übrigen erfindungsgemäßen Ventilsysteme 1a bis 1f übertragbar, so dass auf eine gesonderte Erläuterung dieser Merkmale im Zusammenhang mit diesen Ausführungsformen verzichtet wird.

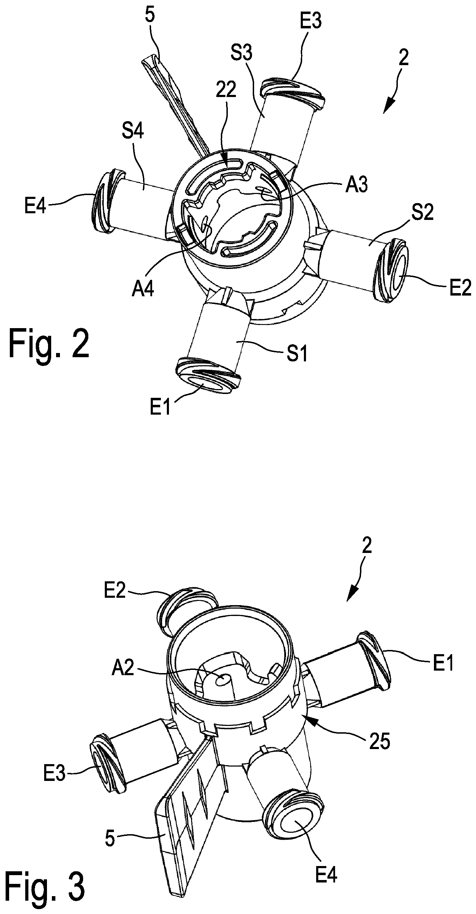

Wie insbesondere anhand der Fig. 2 bis 4 ersichtlich ist, weist das Ventilgehäuse 2 mehrere Medikamenteneinlässe E1 bis E4 auf. Die Medikamenteneinlässe E1 bis E4 sind zur Aufnahme jeweils einer Medikamentenflüssigkeit vorgesehen und fluidleitend mit jeweils einem auf einer inneren Gehäuseumfangsfläche 4 des Ventilgehäuses angeordneten Medikamentenauslass A1 bis A4 verbunden. Die Medikamenteneinlässe E1 bis E4 sind an radial von dem Grundkörper des Ventilgehäuses 2 abragenden Anschlussstutzen S1 bis S4 in Form von Längsbohrungen angeordnet. Stirnendseitig sind die Anschlussstutzen S1 bis S4 jeweils mit einem nicht näher bezeichneten Anschlussgewinde zur Verbindung mit nicht näher bezeichneten Schlauchleitungen für die Zuleitung der jeweiligen Medikamentenflüssigkeit versehen. Alternativ oder zusätzlich zu dem Anschlussgewinde können die Anschlussstutzen S1 bis S4 mit andersartigen Anschlussgeometrien oder mit angeklebten Schlauchabschnitten versehen sein. Das Ventilgehäuse 2 weist zudem ein radial von dem Grundkörper des Ventilgehäuses 2 abragendes Halteelement 5 auf. Zwischen den beiden Stirnendbereichen des Ventilgehäuses 2 erstreckt sich eine im Wesentlichen kreiszylindrisch ausgebildete Durchgangsbohrung 6. Diese Durchgangsbohrung 6 ist zur zumindest abschnittsweisen Aufnahme des Ventilkörpers 3 vorgesehen. Insbesondere zu diesem Zweck kann ein Axialabschnitt der Durchgangsbohrung 6 ein erstes Durchmessermaß und ein weiterer Axialabschnitt ein zweites Durchmessermaß aufweisen, so dass die Durchgangsbohrung 6 gleichsam gestuft sein kann.

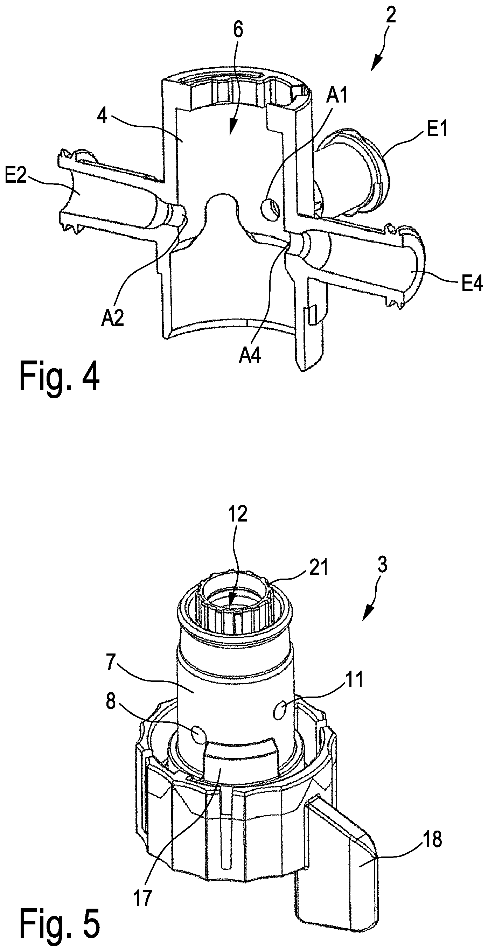

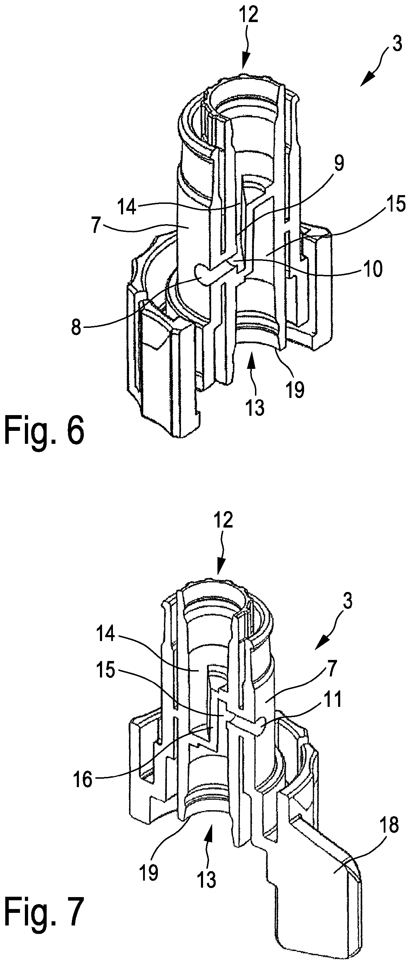

Der Ventilkörper 3 ist insbesondere anhand der Fig. 5 bis 7 näher ersichtlich und weist eine äußere Körperumfangsfläche 7 auf, die zur Aufnahme in der Durchgangsbohrung 6 des Ventilgehäuses 2 vorgesehen ist. Der Ventilkörper 3 weist einen auf der äußeren Körperumfangsfläche 7 angeordneten Spülauslass 8 auf. Der Spülauslass 8 ist fluidleitend mit einem auf einer inneren Körperfläche 9 angeordneten Spüleinlass 10 verbunden. Insoweit weist die fluidleitende Verbindung zwischen dem Spüleinlass 10 und dem Spülauslass 8 die Form einer Querbohrung auf, die sich zumindest abschnittsweise im Wesentlichen in radialer Richtung durch einen Wandbereich des Ventilkörpers 3 erstreckt. In einem oberen Bereich des Ventilkörpers 3 erstreckt sich ein Zuleitungskanal 12, der im Wesentlichen axial zur Rotationsachse des Ventilkörpers 3 orientiert und über den Spüleinlass 10 fluidleitend mit dem Spülauslass 8 verbunden ist. In einem unteren Bereich des Ventilkörpers ist ein Ableitungskanal 13 vorgesehen, der im Wesentlichen axial zur Rotationsachse des Ventilkörpers 3 erstreckt und fluidleitend mit dem Durchlass 11 verbunden ist. Der untere Stirnendbereich des Ableitungskanals 13 bildet den Ventilauslass 19 des Ventilsystems 1. Durch einen zwischen dem Zuleitungskanal 12 und dem Ableitungskanal 13 angeordneten Wandbereich des Ventilkörpers 3 ist eine direkte fluidleitende Verbindung zwischen diesen unterbunden. Der Spülauslass 8 ist in axialer Richtung des Ventilkörpers 3, wie anhand der Fig. 6 und 7 ersichtlich ist, unterhalb des Durchlasses 11 angeordnet. Weiterhin weist der Ventilkörper 3 einen ersten Überleitungskanal 14 auf, der den Spüleinlass 10 fluidleitend mit dem Zuleitungskanal 12 verbindet. Zudem ist ein zweiter Überleitungskanal 15 vorgesehen, der den Durchlass 11 fluidleitend mit dem Ableitungskanal 13 verbindet. Die beiden Überleitungskanäle 14 und 15 sind im Wesentlichen in axialer Richtung des Ventilkörpers 3 erstreckt und verlaufen zumindest abschnittsweise in axialer Richtung nebeneinander.

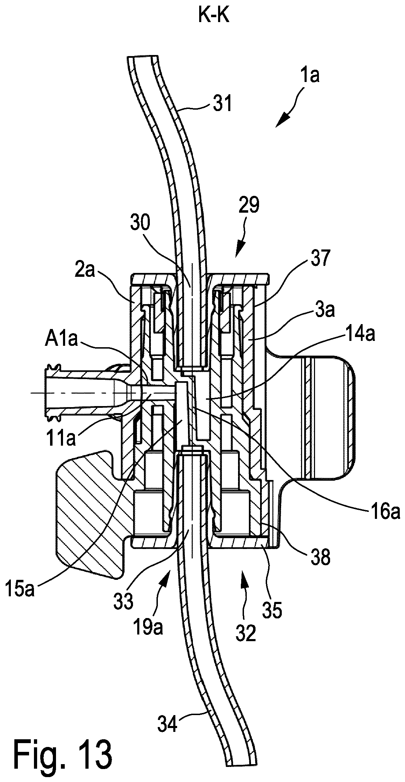

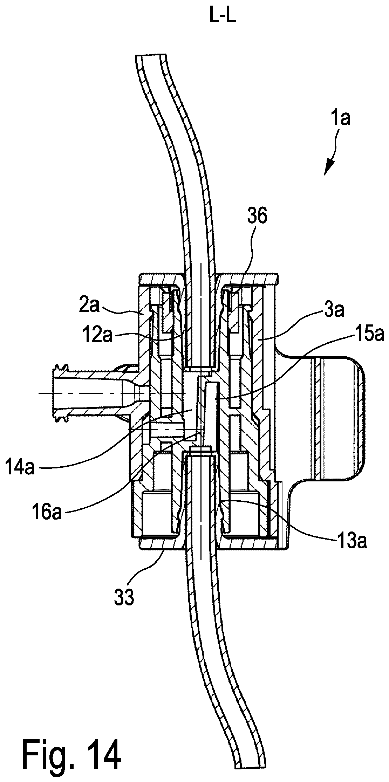

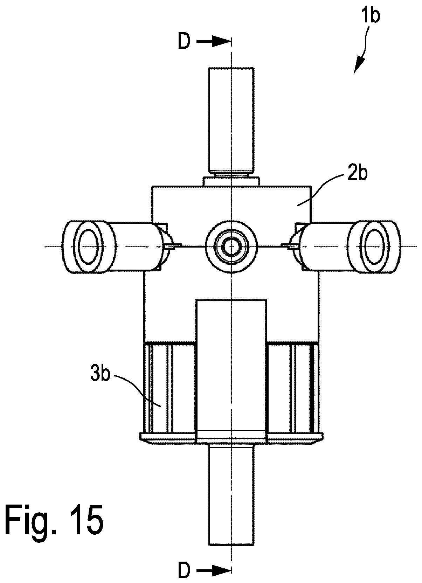

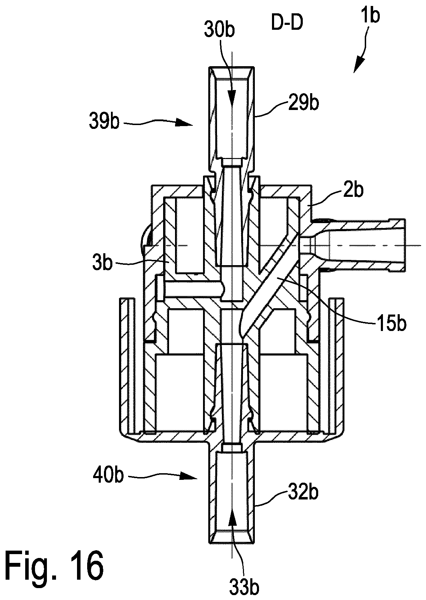

Insbesondere bei dem Ventilsystem 1 sowie bei dem Ventilsystem 1a (Fig. 13, 14) sind der erste Überleitungskanal 14, 14a und der zweite Überleitungskanal 15, 15a zueinander parallel erstreckt und durch eine im Wesentlichen axial verlaufende Trennwand 16, 16a voneinander getrennt. Bei dem Ventilsystem 1b nach den Fig. 15 und 16 sind der erste Überleitungskanal 14b und der zweite Überleitungskanal 15b hingegen zu der Rotationsachse des Ventilkörpers 3b sowie zueinander schräg, im Sinne von nicht zueinander parallel, erstreckt.

Der Ventilkörper 3 weist zudem eine von der äußeren Körperumfangsfläche 7 in radialer Richtung erstreckte Abragung 17 auf, die sich in tangentialer Richtung entlang eines Abschnitts der äußeren Körperumfangsfläche 7 zwischen dem Spülauslass 8 und dem Durchlass 11 erstreckt. An einem unteren Stirnendbereich des Ventilkörpers 3 ist zudem ein sowohl in axialer als auch in radialer Richtung abragendes Handhabungselement 18 vorgesehen.

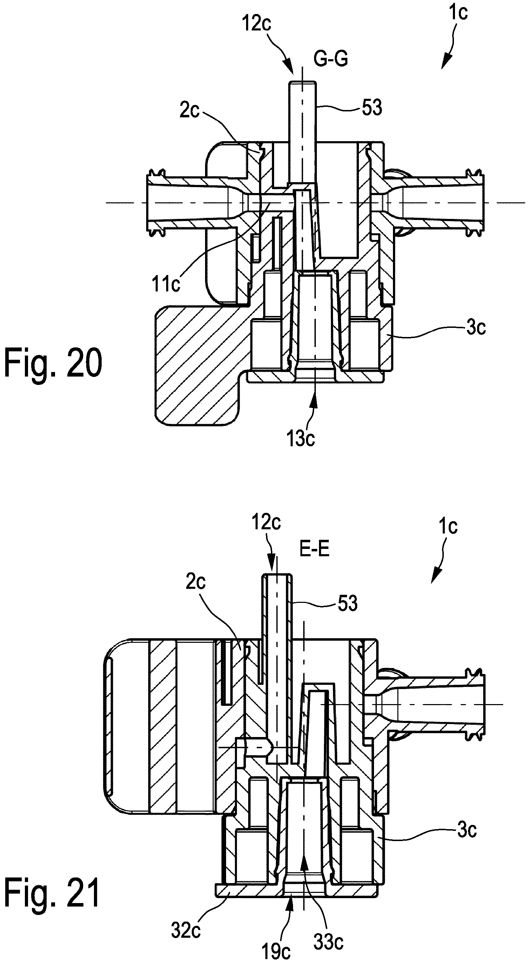

In einem in dem Ventilgehäuse 2 angeordneten Zustand ist der Ventilkörper 3 relativ zu der inneren Gehäuseumfangsfläche 4 in mehrere Medikamentenstellungen sowie wenigstens eine Spülstellung rotierbar. Vorteilhafterweise kann der Ventilkörper 3 zumindest abschnittsweise mittels einer Press- oder Übergangspassung mit dem Ventilgehäuse 2 zusammengefügt sein, so dass ein Fluidaustritt an hierfür nicht vorgesehenen Stellen des Ventilsystems 1 vermieden wird. In einer Medikamentenstellung, wie sie beispielsweise anhand Fig. 13 für das Ventilsystem 1a ersichtlich ist, ist der Durchlass 11a fluidleitend mit einem der Medikamentenauslässe A1a verbunden, so dass die jeweilige Medikamentenflüssigkeit durch den in dem Ventilkörper 3a angeordneten Ventilauslass 19a leitbar ist. In analoger Weise ist eine solche Medikamentenstellung im Zusammenhang mit dem erfindungsgemäßen Ventilsystem 1b nach Fig. 16 und dem Ventilsystem 1c nach Fig. 20 dargestellt. Diese Funktionalität ist insoweit in analoger Weise auf die übrigen erfindungsgemäßen Ventilsysteme 1, 1d bis 1f übertragbar, so dass sich eine gesonderte Erläuterung im Zusammenhang mit diesen Ausführungsformen erübrigt.

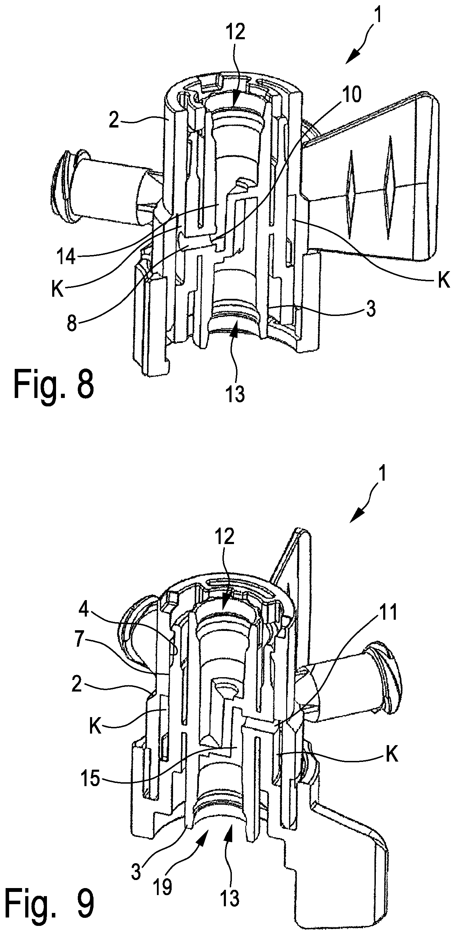

Die Ventilkörper 3 bis 3f der erfindungsgemäßen Ventilsysteme 1 bis 1f sind zudem jeweils in wenigstens eine Spülstellung rotierbar. Eine solche ist im Zusammenhang mit dem Ventilsystem 1 anhand der Fig. 8 und 9 ersichtlich. In dieser Rotationsstellung des Ventilkörpers 3 ist eine fluidleitende Verbindung zwischen dem Zuleitungskanal 12 und dem Ableitungskanal 13 und insoweit mit dem stirnendseitig an dem einem an dem Ventilkörper 3 angeordneten Ventilauslass 19 hergestellt. Diese fluidleitende Verbindung verläuft ausgehend von dem Zuleitungskanal 12 durch den ersten Überleitungskanal 14 in den Spüleinlass 10, von diesem durch den Spülauslass 8 in einen Spülkanal K, der sich ringförmig zwischen der inneren Gehäuseumfangsfläche 4 und der äußeren Körperumfangsfläche 7 erstreckt, weiter in den Durchlass 11, von diesem in den zweiten Überleitungskanal 15 in den Ableitungskanal 13 und den Ventilauslass 19. Auf diese Weise sind etwaige Medikamentenrückstände aus dem Durchlass 11 und dem Ventilauslass sowie aus dem zweiten Überleitungskanal 15 und dem Ableitungskanal 13 spülbar. Hierbei durchströmt die Spülflüssigkeit den Ventilkörper 3 bis 3f in radialer Richtung von innen nach außen gerichtet zwischen dem Spüleinlass 10 und dem Spülauslass 8.

Die radiale Abragung 17 des Ventilkörpers 3 ist dabei derart an der Körperumfangsfläche 7 angeordnet, dass ein Strömungshindernis in dem Spülkanal K ausgebildet ist. Dieses Strömungshindernis verschließt demnach den Querschnitt eines nicht näher ersichtlichen Spülkanalabschnitts zumindest teilweise und erlaubt derart eine funktionsgerechte Fluidführung längs des Spülkanals K.

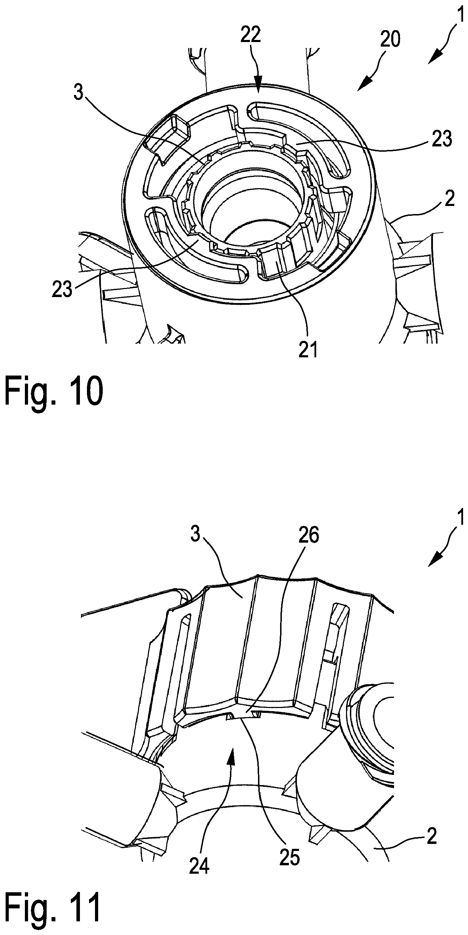

Das Ventilsystem 1 weist ferner eine erste Rastverbindung 20 zwischen dem Ventilgehäuse 2 und dem Ventilkörper 3 zum rotatorischen Festlegen des Ventilkörpers 3 an dem Ventilgehäuse 2 auf. Wie insbesondere anhand Fig. 10 ersichtlich ist, ist die erste Rastverbindung 20 an dem oberen Stirnendbereich des Ventilsystems 1 ausgebildet und umfasst eine an einer äußeren Umfangsfläche des Ventilkörpers 3 ausgebildete Profilierung 21. Die Profilierung 21 ist nach Art einer Verzahnung gestaltet und steht in einer Rastverbindung mit einer an einer innenliegenden Umfangsfläche des Ventilgehäuses 2 angeordneten und komplementär ausgebildeten Profilierung 22. Die Profilierung 22 umfasst zwei Rastnasen 23, die an in Umfangsrichtung gegenüberliegenden Seiten der Profilierung 21 eingreifen. Die Rastnasen 23 können vorteilhafterweise elastisch deformierbar ausgestaltet sein. Durch die erste Rastverbindung 20 wird eine definierte rotatorische Ausrichtung des Ventilkörpers 3 relativ zu dem Ventilgehäuse 2, insbesondere in der Spülstellung und/oder der jeweiligen Medikamentenstellung ermöglicht.

Das Ventilsystem 1 weist zudem eine zweite Rastverbindung 24 auf (Fig. 11). Die zweite Rastverbindung 24 ist in einem unteren Bereich des Ventilsystems 1 zwischen dem Ventilkörper 3 und dem Ventilgehäuse 2 ausgebildet und umfasst eine an einer außenliegenden Gehäuseumfangsfläche des Ventilgehäuses 2 ausgebildete Profilierung 25 sowie eine an einer innenliegenden Umfangsfläche des Ventilkörpers ausgebildete Profilierung 26. Vorzugsweise ist die zweite Profilierung 24 derart ausgebildet, dass der Ventilkörper 3 lediglich in eine Drehrichtung um die Rotationsachse rotierbar ist.

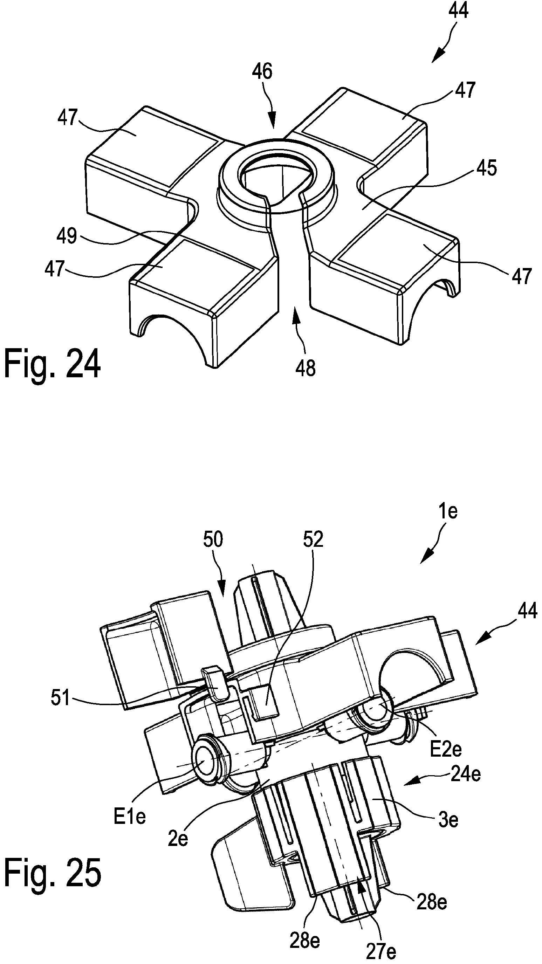

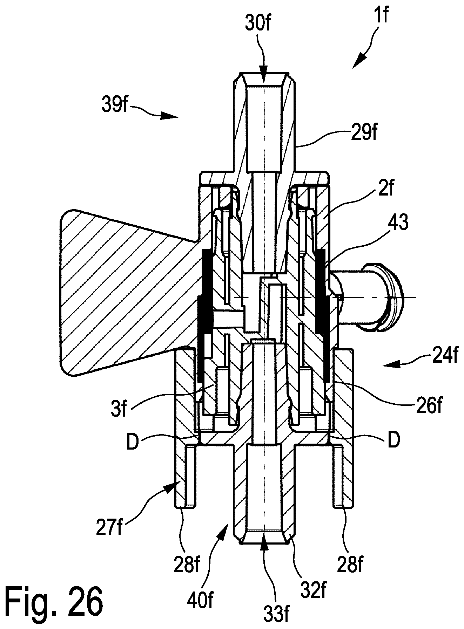

Der strukturelle sowie funktionelle Aufbau der anhand des Ventilsystems 1 erläuterten ersten und zweiten Rastverbindung 20, 24 ist ohne weiteres in analoger Weise auf die übrigen erfindungsgemäßen Ventilsysteme 1a bis 1f übertragbar. Die Ventilsysteme 1e und 1f nach der Fig. 25 bzw. 26 weisen insoweit eine zweite Profilierung 24e, 24f auf, die jeweils mit einem an dem Ventilkörper 3e, 3f angeordneten Entriegelungselement 27e, 27f in einer Wirkverbindung steht. Die Entriegelungselemente 27e, 27f umfassen jeweils zwei in axialer Richtung von dem Ventilkörper 3e, 3f abragende Betätigungsschenkel 28e, 28f. Durch ein Zusammendrücken dieser Betätigungsschenkel 28e, 28f ist die Profilierung 26e, 26f der zweiten Rastverbindung 24e, 24f über einen Drehpunkt D und insoweit gleichsam hebelartig elastisch aufweitbar. Durch dieses elastische Aufweiten kann die zweite Rastverbindung 24e, 24f gelöst und der Ventilkörper 3e, 3f rotatorisch relativ zu dem Ventilgehäuse 2e, 2f positioniert werden.

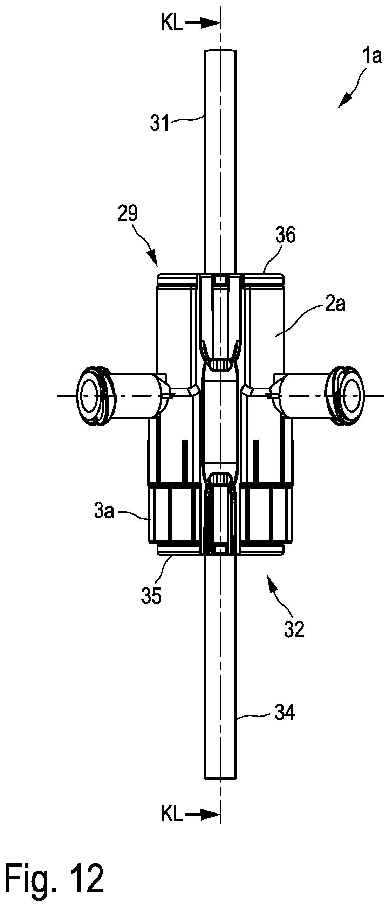

Das Ventilsystem 1a nach den Fig. 12 bis 14 weist zudem eine erste Anschlusshülse 29 mit einer Längsbohrung 30 zur fluiddichten Aufnahme einer Spülflüssigkeitszuleitung 31 auf. Die erste Anschlusshülse 29 ist an ihrem Außenumfang in radialer Richtung in dem Zuleitungskanal 12a des Ventilkörpers 3a festgelegt. Die Innenumfangsfläche der ersten Anschlusshülse 29 umschließt einen Abschnitt der als Schlauchleitung ausgebildeten Spülflüssigkeitszuleitung 31. Die erste Anschlusshülse 29 ist zudem stirnseitig an dem Ventilkörper 3a festgelegt und koaxial zu dessen Rotationsachse ausgerichtet sowie in dem Zuleitungskanal 12a des Ventilkörpers 3a rotierbar. Einem ungewollten Verdrehen des Ventilkörpers 3a durch eine Rotation der Spülflüssigkeitszuleitung 31 und umgekehrt wird durch die derartige Anordnung der ersten Anschlusshülse 29 entgegengewirkt.

Zudem ist eine zweite Anschlusshülse 32 mit einer Längsbohrung 33 zur fluiddichten Aufnahme einer Ventilableitung 34 vorgesehen. Die äußere Umfangsfläche der zweiten Anschlusshülse 32 ist in radialer Richtung in dem Ableitungskanal 13a des Ventilkörpers 3a festgelegt. Die in Form einer Schlauchleitung ausgebildete Ventilableitung 34 ist in die Längsbohrung 33 der zweiten Anschlusshülse 32 eingesteckt und insoweit längs eines Abschnitts ihrer Schlauchmantelfläche in radialer Richtung an der Innenumfangsfläche der zweiten Anschlusshülse 32 festgelegt. Die Längsbohrung 33 ist fluidleitend mit dem Durchlass 11a des Ventilkörpers 3a verbunden. Die zweite Anschlusshülse 32 ist stirnseitig und koaxial zu der Rotationsachse des Ventilkörpers 3a an diesem festgelegt. Insoweit ist die zweite Anschlusshülse 32 in dem Ableitungskanal 13a rotierbar.

Sowohl die erste Anschlusshülse 29 als auch die zweite Anschlusshülse 32 weisen jeweils eine Abdeckplatte 35 bzw. 36 auf. Diese Abdeckplatten 35, 36 sind jeweils derart angeordnet, dass sie einen zwischen dem Ventilkörper 3a und dem Ventilgehäuse 2a ausgebildeten Radialspalt 37 bzw. 38 stirnseitig abdecken. Alternativ oder zusätzlich können die Anschlusshülsen 29 und 32 jeweils rotationsfest an dem Ventilgehäuse 2a festgelegt sein.

Die Ventilsysteme 1b sowie 1f sehen jeweils eine erste Anschlusshülse 29b, 29f vor. Die erste Anschlusshülse 29b, 29f weist jeweils einen Verlängerungsbereich 39b, 39f auf, der sich in axialer Richtung über den Stirnendbereich des Ventilkörpers 3b, 3f erstreckt. In ähnlicher Weise ist die jeweilige zweite Anschlusshülse 32b, 32f mit einem unteren Verlängerungsbereich 40b bzw. 40f ausgestattet.

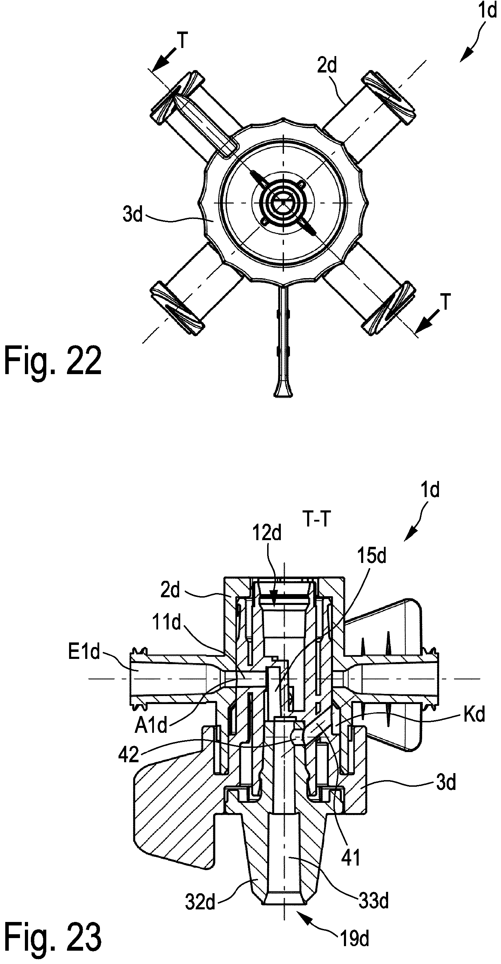

Bei dem erfindungsgemäßen Ventilsystem 1d nach den Fig. 22 und 23 ist zudem vorgesehen, dass der Ventilkörper 3d einen Verbindungskanal 41 aufweist. In der anhand Fig. 23 ersichtlichen Medikamentenstellung des Ventilsystems 1d ist der Verbindungskanal 41 fluidleitend mit dem Spülkanal Kd verbunden. Zudem weist die zweite Anschlusshülse 32d eine von der Längsbohrung 33d in radialer Richtung abzweigende Querbohrung 42 auf. Die zweite Anschlusshülse 32 ist koaxial zu der Rotationsachse des Ventilkörpers 3d an diesem festgelegt und insoweit relativ zu diesem rotierbar. In der anhand Fig. 23 ersichtlichen Funktionsstellung des Ventilsystems 1d, der sogenannten Back-Priming-Stellung, ist die Querbohrung 42 mit dem Verbindungskanal 41 fluidleitend verbunden. Durch diese Ausgestaltung des Ventilsystems 1d ist eine Rückspülung des Medikamenteneinlasses E1d mit Spülflüssigkeit möglich. Zu diesem Zweck wird zunächst der Ventilauslass 19d oder eine die anhand Fig. 23 nicht näher ersichtliche Ventilableitung verschlossen, beispielsweise mit einer Klemme. Die Spülflüssigkeit kann dann über die fluidleitende Verbindung zwischen dem Zuleitungskanal 12d, dem Spüleinlass, dem Spülauslass, dem Spülkanal K, dem Verbindungskanal 41 der Querbohrung 42, der Längsbohrung 33d, dem zweiten Überleitungskanal 15d und dem Durchlass 11d über den Medikamentenauslass A1d in den Medikamenteneinlass E1d gelangen. Der Medikamenteneinlass E1d kann derart mit Spülflüssigkeit rückgespült werden. Durch eine rotatorische Verlagerung der zweiten Anschlusshülse 32d zu dem Ventilkörper 3d kann die fluidleitende Verbindung zwischen der Querbohrung 42 und dem Verbindungskanal 41 aufgehoben werden. Hiernach befindet sich das Ventilsystem 1d in einer Medikamentenstellung, in welcher Medikamentenflüssigkeit über den Medikamenteneinlass E1d durch den Medikamentenauslass A1d, den Durchlass 11d in den zweiten Überleitungskanal 15d und von diesem weiter durch die Längsbohrung 33d bis zu dem von dem Stirnendbereich der zweiten Anschlusshülse 32d gebildeten Ventilauslass 19d gelangen kann.

Um eine verbesserte Abdichtung zwischen dem Ventilkörper 3f und dem Ventilgehäuse 2f zu gewährleisten, sieht das erfindungsgemäße Ventilsystem 1f ein Dichtelement 43 vor. Das Dichtelement 43 ist in radialer Richtung zwischen dem Ventilkörper 3f und dem Ventilgehäuse 2f angeordnet und weist eine elastomere Materialzusammensetzung auf.

Das erfindungsgemäße Ventilsystem 1e nach Fig. 25 sieht eine Abdeckvorrichtung 44 vor. Die Abdeckvorrichtung 44 ist anhand Fig. 24 näher ersichtlich und weist einen Grundkörper 45 mit einer durchgehenden Öffnung 46 zur Aufnahme einer Schlauchleitung sowie einer der Anzahl der Medikamenteneinlässe E1e bis E4e entsprechenden Anzahl, hier: vier, sternförmig um den Grundkörper 45 angeordnete Abdeckelemente 47 auf. Die durchgehende Öffnung 46 ist mit einem Radialschlitz 48 versehen, so dass die Abdeckvorrichtung 44 in radialer Richtung über eine etwaig vorhandene Spülflüssigkeitsleitung des Ventilsystems 1e geschoben werden kann. Die Abdeckelemente 47 weisen stirnseitig halbkreisförmige Ausnehmungen auf, die zur Aufnahme von mit den Medikamenteneinlässen E1e bis E4e verbindbaren Medikamentenzuleitungen vorgesehen sind. Wie anhand Fig. 25 ersichtlich ist, sind die Abdeckelemente 47 derart gestaltet und angeordnet, dass die manuelle Zugänglichkeit zu den Medikamenteneinlässen E1e bis E4e eingeschränkt ist. Einem ungewollten Lösen der Medikamentenzuleitungen von den Medikamenteneinlässen E1e bis E4e wird derart entgegengewirkt. Die Abdeckelemente 47 sind jeweils über ein Befestigungsmittel in Form eines Filmscharniers 49 mit dem Grundkörper 45 verbunden und relativ zu diesem verschwenkbar. Insoweit können die Abdeckelemente 47 über die Filmscharniere 49 zum Anschließen von Medikamentenzuleitungen an die jeweiligen Medikamenteneinlässe E1e bis E4e nach oben verschwenkt werden. Nach erfolgtem Anschluss der Medikamentenzuleitungen können die Abdeckelemente 47 dementsprechend nach unten verschwenkt werden. In der nach unten verschwenkten Stellung sind die Abdeckelemente 47 über jeweils ein Verbindungsmittel 50 mit dem Grundkörper 45 verbunden. Die Verbindungsmittel 50 weisen jeweils ein an dem Abdeckelement 47 seitlich angeordnetes Rastelement 51 sowie eine an dem Grundkörper 45 angeordnete Rasttasche 52 auf.

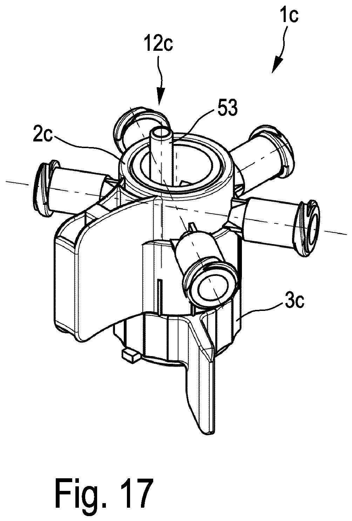

Im Unterschied zu den Ventilsystemen 1 bis 1b, 1d bis 1f sieht das Ventilsystem 1c nach den Fig. 17 bis 21 einen radialen Versatz zwischen dem Zuleitungskanal 12c und dem Ableitungskanal 13c vor. Der Zuleitungskanal 12c ist als in axialer Richtung nach oben erstreckte Hülse 53 an dem Ventilkörper 3c angeordnet. Vorteilhafterweise kann die Hülse 53 einstückig mit dem Ventilkörper 3c ausgebildet sein. Zur Verbindung des Ventilkörpers 3c mit einer nicht näher ersichtlichen Spülflüssigkeitszuleitung wird die Hülse 53 in die Spülflüssigkeitszuleitung eingesteckt. Derart kann eine besonders kurze Bauform des Ventilsystems 1c erreicht werden. Vorteilhafterweise kann die Spülflüssigkeitszuleitung mit der Hülse 53 verklebt werden.

## Patentansprüche

1. Medizinisches Ventilsystem (1 bis 1f) zur Verabreichung von wenigstens zwei Medikamentenflüssigkeiten mit
- einem Ventilgehäuse (2 bis 2f) mit wenigstens zwei Medikamenteneinlässen (E1 bis E4) zur Aufnahme jeweils einer der Medikamentenflüssigkeiten, die fluidleitend mit jeweils einem auf einer inneren Gehäuseumfangsfläche (4) angeordneten Medikamentenauslass (A1 bis A4) verbunden sind,
- einem zumindest abschnittsweise in dem Ventilgehäuse (2 bis 2f) angeordneten Ventilkörper (3 bis 3f), der um eine Rotationachse relativ rotierbar zu der inneren Gehäuseumfangsfläche (4) ist, mit einer äußerem Körperumfangsfläche (7) und einer inneren Körperfläche (9) und einem auf der äußeren Körperumfangsfläche (7) angeordneten Durchlass (11, 11a, 11c, 11d),
- einem Spüleinlass (10) zur Aufnahme einer Spülflüssigkeit der fluidleitend mit einem Spülauslass (8) verbunden ist und
- einem Ventilauslass (19, 19a, 19c, 19d) zur Abgabe der Spülflüssigkeit und/oder wenigstens einer der Medikamentenflüssigkeiten, der fluidleitend mit dem Durchlass (11, 11a, 11c, 11d) verbunden ist,
- wobei der Ventilkörper (3 bis 3f) rotierbar ist in
- wenigstens zwei Medikamentenstellungen, in denen der Durchlass (11, 11a, 11c, 11d) jeweils derart fluidleitend mit einem der Medikamentenauslässe (A1 bis A4) verbunden ist, dass die jeweilige Medikamentenflüssigkeit durch den Ventilauslass (19, 19a, 19c, 19d) leitbar ist, und
- wenigstens eine Spülstellung, in der der Durchlass (11, 11a, 11c, 11d) über einen Spülkanal (K, Ka, Kd) derart fluidleitend mit dem Spülauslass (8) verbunden ist, dass die Spülflüssigkeit durch den Ventilauslass (19, 19a, 19c, 19d) leitbar und derart Rückstände der ersten und/oder zweiten Medikamentenflüssigkeit aus dem Durchlass (11, 11a, 11c, 11d) und dem Ventilauslass (19, 19a, 19c, 19d) spülbar sind,
**dadurch gekennzeichnet, dass** der Spüleinlass (10) und der Ventilauslass (19, 19a, 19c, 19d) an dem Ventilkörper (3 bis 3f) angeordnet sind, wobei der Spüleinlass (10) auf der inneren Körperfläche (9) des Ventilkörpers (3 bis 3f) angeordnet ist und der Spülauslass (8) auf der äußeren Körperumfangsfläche (7) des Ventilkörpers (3 bis 3f) angeordnet ist, so dass die Spülflüssigkeit in der Spülstellung von innen nach außen gerichtet durch den Ventilkörper (3 bis 3f) leitbar ist, und wobei der Ventilkörper (3 bis 3f) einen Zuleitungskanal (12, 12a, 12c) der fluidleitend mit dem Spülauslass (8) verbunden und im Wesentlichen axial zur Rotationsachse des Ventilkörpers (3 bis 3f) erstreckt ist, sowie einen Ableitungskanal (13, 13a, 13c) aufweist, der fluidleitend mit dem Durchlass (11, 11a, 11c, 11d) und dem Ventilauslass (19, 19a, 19c, 19d) verbunden und im Wesentlichen axial zu dem Zuleitungskanal (12, 12a, 12c) erstreckt ist.

2. Medizinisches Ventilsystem (1 bis 1f) nach Anspruch 1, **dadurch gekennzeichnet, dass** der Spülauslass (8) unterhalb des Durchlasses (11, 11a, 11c, 11d) angeordnet ist, wobei der Ventilkörper (3e bis 3f) einen ersten Überleitungskanal (14 bis 14b) aufweist, der den Spüleinlass (10) fluidleitend mit dem Zuleitungskanal (12, 12a, 12c) verbindet, sowie einen zweiten Überleitungskanal (15 bis 15b) aufweist, der den Durchlass (11, 11a, 11c, 11d) fluidleitend mit dem Ableitungskanal (13, 13a, 13c) verbindet, und wobei die Überleitungskanäle in axialer Richtung des Ventilkörpers (3 bis 3f) zumindest abschnittsweise nebeneinander angeordnet sind.

3. Medizinisches Ventilsystem (1, 1a, 1c bis 1f) nach Anspruch 2, **dadurch gekennzeichnet, dass** der erste Überleitungskanal (14, 14a) und der zweite Überleitungskanal (15, 15a) wenigstens abschnittsweise im Wesentlichen zueinander parallel erstreckt und durch eine im Wesentlichen axial erstreckte Trennwand (16, 16a) voneinander getrennt sind.

4. Medizinisches Ventilsystem (1b) nach Anspruch 2, **dadurch gekennzeichnet, dass** der erste Überleitungskanal (14b) und/oder der zweite Überleitungskanal (15b) zu der Rotationsachse schräg erstreckt ist.

5. Medizinisches Ventilsystem (1 bis 1f) nach einem der vorhergehenden Ansprüche, **dadurch gekennzeichnet, dass** der Spülkanal (K, Ka, Kd) ringförmig zwischen der inneren Gehäuseumfangsfläche (4) und der äußeren Körperumfangsfläche (7) ausgebildet ist, insbesondere wobei der Ventilkörper (3 bis 3f) wenigstens eine radiale Abragung (17) aufweist, die derart an der Körperumfangsfläche (7) angeordnet ist, dass wenigstens ein Strömungshindernis in dem Spülkanal (K, Ka, Kd) ausgebildet ist.

6. Medizinisches Ventilsystem (1 bis 1f) nach einem der vorhergehenden Ansprüche, **gekennzeichnet durch** eine erste Rastverbindung (20) zwischen den Ventilgehäuse (2 bis 2f) und dem Ventilkörper (3 bis 3f) zum rotatorischen Festlegen des Ventilkörpers (3 bis 3f) an dem Ventilgehäuse (2 bis 2f), insbesondere **gekennzeichnet durch** eine zweite Rastverbindung (24) zwischen dem Ventilgehäuse (2 bis 2f) und dem Ventilkörper (3 bis 3f), die derart angeordnet ist, dass der Ventilkörper (3 bis 3f) lediglich in eine Drehrichtung um die Rotationsachse rotierbar ist, insbesondere wobei die erste und/oder zweite Rastverbindung (20, 24) jeweils eine/eine an einer Umfangsfläche des Ventilkörpers (3 bis 3f) umlaufend angeordnete Profilierung (21, 26) aufweist.

7. Medizinisches Ventilsystem (1e, 1f) nach Anspruch 6, **dadurch gekennzeichnet, dass** der Ventilkörper (3e, 3f) wenigstens ein manuell betätigbares Entriegelungselement (27e, 27f) aufweist, das derart ausgestaltet ist, dass die Profilierung (21, 26) elastisch radial aufweitbar und derart die erste und/oder zweite Rastverbindung (20, 24) zwischen dem Ventilkörper (3e, 3f) und dem Ventilgehäuse (2e, 2f) lösbar ist.

8. Medizinisches Ventilsystem (1a, 1b, 1f) nach einem der vorhergehenden Ansprüche, **gekennzeichnet durch** eine erste Anschlusshülse (29, 29b, 29f) mit einer Längsbohrung (30, 30b, 30f) zur fluiddichten Aufnahme einer Spülflüssigkeitszuleitung (31), wobei die Längsbohrung (30, 30b, 30f) fluidleitend mit dem Spüleinlass (10) verbunden ist und wobei die erste Anschlusshülse (29, 29b, 29f) derart stirnseitig und koaxial zu der Rotationsachse an dem Ventilkörper (3a, 3b, 3f) festgelegt ist, dass der Ventilkörper (3a, 3b, 3f) relativ rotierbar zu der ersten Anschlusshülse (29, 29b, 29f) ist.

9. Medizinisches Ventilsystem (1a bis 1d, 1f) nach einem der vorhergehenden Ansprüche, **gekennzeichnet durch** eine zweite Anschlusshülse (32, 32b bis 32d, 32f) mit einer Längsbohrung (33, 33b bis 33d, 33f) zur fluiddichten Aufnahme einer Ventilableitung (34), wobei die Längsbohrung (33, 33b bis 33d, 33f) fluidleitend mit dem Durchlass (11) verbunden ist und die zweite Anschlusshülse (32, 32b bis 32d, 32f) derart stirnseitig und koaxial zu der Rotationsachse an dem Ventilkörper (3a bis 3d, 3f) festgelegt ist, dass der Ventilkörper (3a bis 3d, 3f) relativ rotierbar zu der zweiten Anschlusshülse (32, 32b bis 32d, 32f) ist.

10. Medizinisches Ventilsystem (1a) nach Anspruch 8 und/oder 9, **dadurch gekennzeichnet, dass** die erste Anschlusshülse (29) und/oder die zweite Anschlusshülse (32) rotationsfest an dem Ventilgehäuse (3a) befestigt sind/ist, insbesondere wobei die erste und/oder die zweite Anschlusshülse (29, 32) jeweils eine/eine Abdeckplatte (36, 36) aufweisen/aufweist, die derart angeordnet sind/ist, dass jeweils ein/ein zwischen dem Ventilkörper (3a) und dem Ventilgehäuse (2a) ausgebildeter Radialspalt (37, 38) stirnseitig abgedeckt ist.

11. Medizinisches Ventilsystem (1d) nach Anspruch 9, **dadurch gekennzeichnet, dass** der Ventilkörper (3d) einen Verbindungskanal (41) aufweist, der in wenigstens einer der Medikamentenstellungen fluidleitend mit dem Spülkanal (Kd) verbunden ist, und dass die zweite Anschlusshülse (32d) eine von der Außenumfangsfläche der Anschlusshülse (32d) in deren Längsbohrung (33d) reichende Querbohrung (42) aufweist, wobei die zweite Anschlusshülse (32d) ausgehend von der jeweiligen Medikamentenstellung des Ventilkörpers (3d) relativ zu diesem rotierbar ist in eine Back-Primingstellung, in der die Querbohrung (42) fluidleitend mit dem Verbindungskanal (41) verbunden ist.

12. Medizinisches Ventilsystem (1f) nach einem der vorhergehenden Ansprüche, **gekennzeichnet durch** ein zwischen der äußeren Körperumfangsfläche (7) und der inneren Gehäuseumfangsfläche (4) angeordnetes Dichtelement (43) zum Abdichten eines Radialspaltes zwischen dem Ventilkörper (3f) und dem Ventilgehäuse (2f).

13. Medizinisches (1e) Ventilsystem nach einem der vorhergehenden Ansprüche, **gekennzeichnet durch** eine stirnseitig an dem Ventilgehäuse (2e) angeordnete Abdeckvorrichtung (44) mit einem Grundkörper (45) mit einer durchgehenden Öffnung (46) zur Aufnahme einer Schlauchleitung sowie mindestens einem im Bereich mindestens eines der Medikamenteneinlässe (E1e bis E4e) angeordneten Abdeckelement (47) , dass derart gestaltet ist, dass es die manuelle Zugänglichkeit zu dem Medikamenteneinlass (E1e bis E4e) einschränkt, insbesondere wobei das Abdeckelement (47) ein Verbindungsmittel (50) aufweist, das derart gestaltet ist, dass eine Medikamentenzuleitung an dem Abdeckelement (47) formschlüssig arretierbar ist und/oder wobei das Abdeckelement (47) mittels eines Befestigungsmittels (49) relativ verschwenkbar zu dem Grundkörper (45) an diesem befestigt ist, insbesondere mittels eines Filmscharniers (49).

## Claims

1. Medical valve system (1 to 1f) for administering at least two medicament liquids, having
- a valve housing (2 to 2f) with at least two medicament inlets (E1 to E4) which each receive one of the medicament liquids and which are fluidically connected in each case to a medicament outlet (A1 to A4) arranged on an inner circumferential surface (4) of the housing,
- a valve body (3 to 3f) which is arranged at least partially in the valve housing (2 to 2f) and which is rotatable about a rotation axis relative to the inner circumferential surface (4) of the housing, with an outer circumferential surface (7) of the body and an inner body surface (9) and with a passageway (11, 11a, 11c, 11d) arranged on the outer circumferential surface (7) of the body,
- a flushing inlet (10) which receives a flushing liquid and which is fluidically connected to a flushing outlet (8), and
- a valve outlet (19, 19a, 19c, 19d) which discharges the flushing liquid and/or at least one of the medicament liquids and which is fluidically connected to the passageway (11, 11a, 11c, 11d),
- wherein the valve body (3 to 3f) is rotatable to
- at least two medicament positions, in which the passageway (11, 11a, 11c, 11d) is in each case fluidically connected to one of the medicament outlets (A1 to A4) in such a way that the respective medicament liquid can be conveyed through the valve outlet (19, 19a, 19c, 19d), and
- at least one flushing position, in which the passageway (11, 11a, 11c, 11d) is fluidically connected via a flushing channel (K, Ka, Kd) to the flushing outlet (8) in such a way that the flushing liquid can be conveyed through the valve outlet (19, 19a, 19c, 19d) and so residues of the first and/or second medicament liquid can be flushed out of the passageway (11, 11a, 1c, 11d) and the valve outlet (19, 19a, 19c, 19d),
**characterized in that** the flushing inlet (10) and the valve outlet (19, 19a, 19c, 19d) are arranged on the valve body (3 to 3f), wherein the flushing inlet (10) is arranged on the inner body surface (9) of the valve body (3 to 3f), and the flushing outlet (8) is arranged on the outer circumferential surface (7) of the valve body (3 to 3f), such that the flushing liquid, in the flushing position, can be conveyed through the valve body (3 to 3f) from the inside outwards, and wherein the valve body (3 to 3f) has an admission channel (12, 12a, 12c) which is fluidically connected to the flushing outlet (8) and extends substantially axially with respect to the rotation axis of the valve body (3 to 3f), and also an output channel (13, 13a, 13c) which is fluidically connected to the passageway (11, 11a, 11c, 11d) and the valve outlet (19, 19a, 19c, 19d) and extends substantially axially with respect to the admission channel (12, 12a, 12c).

2. Medical valve system (1 to 1f) according to Claim 1, **characterized in that** the flushing outlet (8) is arranged below the passageway (11, 11a, 11c, 11d), wherein the valve body (3e to 3f) has a first transfer channel (14 to 14b), which fluidically connects the flushing inlet (10) to the admission channel (12, 12a, 12c), and a second transfer channel (15 to 15b), which fluidically connects the passageway (11, 11a, 11c, 11d) to the output channel (13, 13a, 13c), and wherein the transfer channels are arranged at least in part next to each other in the axial direction of the valve body (3 to 3f).

3. Medical valve system (1, 1a, 1c to 1f) according to Claim 2, **characterized in that** the first transfer channel (14, 14a) and the second transfer channel (15, 15a) extend substantially parallel to each other at least in part and are separated from each other by a substantially axially extending partition wall (16, 16a).

4. Medical valve system (1b) according to Claim 2, **characterized in that** the first transfer channel (14b) and/or the second transfer channel (15b) extends at an inclination with respect to the rotation axis.

5. Medical valve system (1 to 1f) according to one of the preceding claims, **characterized in that** the flushing channel (K, Ka, Kd) is formed annularly between the inner circumferential surface (4) of the housing and the outer circumferential surface (7) of the body, in particular wherein the valve body (3 to 3f) has at least one radial protrusion (17), which is arranged on the circumferential surface (7) of the body in such a way that at least one flow obstacle is formed in the flushing channel (K, Ka, Kd).

6. Medical valve system (1 to 1f) according to one of the preceding claims, **characterized by** a first latch connection (20) between the valve housing (2 to 2f) and the valve body (3 to 3f), for securing the valve body (3 to 3f) in terms of rotation on the valve housing (2 to 2f), in particular **characterized by** a second latch connection (24) between the valve housing (2 to 2f) and the valve body (3 to 3f), which is arranged in such a way that the valve body (3 to 3f) is rotatable only in one direction of rotation about the rotation axis, in particular wherein the first and/or second latch connection (20, 24) respectively have/has a profile (21, 26) arranged peripherally on a circumferential surface of the valve body (3 to 3f).

7. Medical valve system (1e, 1f) according to Claim 6, **characterized in that** the valve body (3e, 3f) has at least one manually actuatable unlocking element (27e, 27f), which is configured in such a way that the profile (21, 26) is elastically radially expansible and in such a way that the first and/or second latch connection (20, 24) between the valve body (3e, 3f) and the valve housing (2e, 2f) is releasable.

8. Medical valve system (1a, 1b, 1f) according to one of the preceding claims, **characterized by** a first attachment sleeve (29, 29b, 29f) with a longitudinal bore (30, 30b, 30f) for receiving a flushing liquid admission line (31) in a fluid-tight manner, wherein the longitudinal bore (30, 30b, 30f) is fluidically connected to the flushing inlet (10), and wherein the first attachment sleeve (29, 29b, 29f) is secured on the valve body (3a, 3b, 3f) at the end face and coaxially with respect to the rotation axis in such a way that the valve body (3a, 3b, 3f) is rotatable relative to the first attachment sleeve (29, 29b, 29f).

9. Medical valve system (1a to 1d, 1f) according to one of the preceding claims, **characterized by** a second attachment sleeve (32, 32b to 32d, 32f) with a longitudinal bore (33, 33b to 33d, 33f) for receiving a valve output line (34) in a fluid-tight manner, wherein the longitudinal bore (33, 33b to 33d, 33f) is fluidically connected to the passageway (11), and the second attachment sleeve (32, 32b to 32d, 32f) is secured on the valve body (3a to 3d, 3f) at the end face and coaxially with respect to the rotation axis in such a way that the valve body (3a to 3d, 3f) is rotatable relative to the second attachment sleeve (32, 32b to 32d, 32f).

10. Medical valve system (1a) according to Claim 8 and/or 9, **characterized in that** the first attachment sleeve (29) and/or the second attachment sleeve (32) are/is secured in a rotationally fixed manner on the valve housing (3a), in particular wherein the first and/or the second attachment sleeve (29, 32) have/has a respective cover plate (36, 36), the latter being arranged in such a way that a radial gap (37, 38) formed between the valve body (3a) and the valve housing (2a) is in each case covered at the end face.

11. Medical valve system (1d) according to Claim 9, **characterized in that** the valve body (3d) has a connection channel (41) which is fluidically connected to the flushing channel (Kd) in at least one of the medicament positions, and **in that** the second attachment sleeve (32d) has a transverse bore (42) which reaches from the outer circumferential surface of the attachment sleeve (32d) into the longitudinal bore (33d) thereof, wherein the second attachment sleeve (32d), proceeding from the respective medicament position of the valve body (3d), is rotatable relative to the latter into a back-priming position in which the transverse bore (42) is fluidically connected to the connection channel (41).

12. Medical valve system (1f) according to one of the preceding claims, **characterized by** a sealing element (43) which is arranged between the outer circumferential surface (7) of the body and the inner circumferential surface (4) of the housing and serves to seal a radial gap between the valve body (3f) and the valve housing (2f).

13. Medical (1e) valve system according to one of the preceding claims, **characterized by** a cover device (44) arranged on the end face of the valve housing (2e) and having a main body (45) with a through-opening (46) for receiving a hose line and with at least one cover element (47) arranged in the region of at least one of the medicament inlets (E1e to E4e), which cover element (47) is configured in such a way that it limits the manual accessibility to the medicament inlet (E1e to E4e), in particular wherein the cover element (47) has a connection means (50) which is configured in such a way that a medicament admission line can be locked with form-fit engagement on the cover element (47), and/or wherein the cover element (47) is secured on the main body (45), in a manner pivotable relative to the latter, by means of a securing means (49), in particular by means of a film hinge (49).

## Revendications

1. Système de vannes médical (1 à 1f) servant à administrer au moins deux fluides médicamenteux avec :
- un carter de vanne (2 à 2f) équipé d'au moins deux admissions de médicament (E1 à E4) destinées à recevoir respectivement un des fluides médicamenteux reliés en écoulement de fluide à respectivement une sortie de médicament (A1 à A4) disposée sur une surface périphérique intérieure de carter (4) ;
- un corps de vanne (3 à 3f) disposé au moins en partie dans le carter de vanne (2 à 2f) et pouvant tourner autour d'un axe de rotation par rapport à la surface périphérique intérieure de carter (4), avec une surface périphérique extérieure de corps (7) et une surface intérieure de corps (9) et avec un passage (11, 11a, 11c, 11d) disposé sur la surface périphérique extérieure de corps (7) ;
- avec une admission de rinçage (10) destinée à recevoir un fluide de rinçage relié en écoulement de fluide à une sortie de rinçage (8) ; et
- une sortie de vanne (19, 19a, 19c, 19d) servant à dispenser le fluide de rinçage et/ou au moins un des fluides médicamenteux relié en écoulement de fluide avec le passage (11, 11a, 11c, 11d) ;
- le corps de vanne (3 à 3f) pouvant tourner dans :
- au moins deux positions de médicament dans lesquelles le passage (11, 11a, 11c, 11d) est respectivement relié en écoulement de fluide à une des sorties de médicament (A1 à A4), de telle sorte que le fluide médicamenteux respectif puisse être guidé à travers la sortie de vanne (19, 19a, 19c, 19d) ; et
- au moins une position de rinçage dans laquelle le passage (11, 11a, 11c, 11d) est relié en écoulement de fluide à la sortie de rinçage (8), via un canal de rinçage (K, Ka, Kd), de telle sorte que le fluide de rinçage puisse être guidé à travers la sortie de vanne (19, 19a, 19c, 19d) et que les résidus du premier et/ou du deuxième fluide médicamenteux puissent être rincés en sortant du passage (11, 11a, 11c, 11d) et de la sortie de vanne (19, 19a, 19c, 19d) ;
**caractérisé en ce que** l'admission de rinçage (10) et la sortie de vanne (19, 19a, 19c, 19d) sont disposées au niveau du corps de vanne (3 à 3f), l'admission de rinçage (10) étant disposée sur la surface intérieure de corps (9) du corps de vanne (3 à 3f) et la sortie de rinçage (8) étant disposée sur la surface périphérique extérieure de corps (7) du corps de vanne (3 à 3f), de sorte que le fluide de rinçage puisse être guidé dans la position de rinçage en étant orienté de l'intérieur vers l'extérieur à travers le corps de vanne (3 à 3f) et le corps de vanne (3 à 3f) comportant un canal d'amenée (12, 12a, 12c) relié en écoulement de fluide à la sortie de rinçage (8) et s'étendant pour l'essentiel dans le plan axial par rapport à l'axe de rotation du corps de vanne (3 à 3f), ainsi qu'un canal d'évacuation (13, 13a, 13c) relié en écoulement de fluide au passage (11, 11a, 11c, 11d) et à la sortie de vanne (19, 19a, 19c, 19d) et s'étendant pour l'essentiel dans le plan axial par rapport au canal d'amenée (12, 12a, 12c).

2. Système de vannes médical (1 à 1f) selon la revendication 1, **caractérisé en ce que** la sortie de rinçage (8) est disposée en dessous du passage (11, 11a, 11c, 11d), le corps de vanne (3e à 3f) comportant un premier canal de transfert (14 à 14b) reliant en écoulement de fluide l'admission de rinçage (10) au canal d'amenée (12, 12a, 12c), ainsi qu'un deuxième canal de transfert (15 à 15b) reliant en écoulement de fluide le passage (11, 11a, 11c, 11d) au canal d'évacuation (13, 13a, 13c) et les canaux de transfert étant disposés au moins en partie côte à côte dans la direction axiale du corps de vanne (3 à 3f).

3. Système de vannes médical (1, 1a, 1c à 1f) selon la revendication 2, **caractérisé en ce que** le premier canal de transfert (14, 14a) et le deuxième canal de transfert (15, 15a) s'étendent au moins en partie pour l'essentiel parallèlement l'un par rapport à l'autre et sont séparés l'un de l'autre par une paroi de séparation (16, 16a) s'étendant pour l'essentiel dans le plan axial.

4. Système de vannes médical (1b) selon la revendication 2, **caractérisé en ce que** le premier canal de transfert (14b) et/ou le deuxième canal de transfert (15b) s'étendent de façon inclinée par rapport à un axe de rotation.

5. Système de vannes médical (1 à 1f) selon l'une quelconque des revendications précédentes, **caractérisé en ce que** le canal de rinçage (K, Ka, Kd) est réalisé en forme d'anneau entre la surface périphérique intérieure de carter (4) et la surface périphérique extérieure de corps (7), le corps de vanne (3 à 3f) comportant au moins une saillie radiale (17) qui est disposée de telle sorte au niveau de la surface périphérique de corps (7) qu'au moins un obstacle d'écoulement est réalisé dans le canal de rinçage (K, Ka, Kd).

6. Système de vannes médical (1 à 1f) selon l'une quelconque des revendications précédentes, **caractérisé par** la présence d'une première liaison d'arrêt (20) entre le carter de vanne (2 à 2f) et le corps de vanne (3 à 3f) servant à fixer en rotation le corps de vanne (3 à 3f) au niveau du carter de vanne (2 à 2f), notamment **caractérisé par** la présence d'une deuxième liaison d'arrêt (24) entre le carter de vanne (2 à 2f) et le corps de vanne (3 à 3f) qui est disposée de telle sorte que le corps de vanne (3 à 3f) puisse tourner seulement dans une direction de rotation autour de l'axe de rotation, la première et/ou deuxième liaison d'arrêt (20, 24) comportant notamment respectivement un profilage (21, 26) disposé autour au niveau d'une surface périphérique du corps de vanne (3 à 3f).

7. Système de vannes médical (1e, 1f) selon la revendication 6, **caractérisé en ce que** le corps de vanne (3e, 3f) comporte au moins un élément de déverrouillage (27e, 27f) pouvant être déverrouillé manuellement qui est configuré de telle sorte que le profilage (21, 26) puisse être élargi de façon élastique dans le plan radial et de telle sorte que la première et/ou deuxième liaison d'arrêt (20, 24) entre le corps de vanne (3e, 3f) et le carter de vanne (2e, 2f) soit amovible.

8. Système de vannes médical (1a, 1b, 1f) selon l'une quelconque des revendications précédentes, **caractérisé par** la présence d'un premier manchon de raccordement (29, 29b, 29f) avec un alésage longitudinal (30, 30b, 30f) permettant de recevoir de façon étanche aux fluides une conduite d'amenée de fluide de rinçage (31), l'alésage longitudinal (30, 30b, 30f) étant relié en écoulement de fluide à l'admission de rinçage (10) et le premier manchon de raccordement (29, 29b, 29f) étant fixé de telle sorte sur le côté avant et dans le plan coaxial par rapport à l'axe de rotation, au niveau du corps de vanne (3a, 3b, 3f), que le corps de vanne (3a, 3b, 3f) puisse tourner par rapport au premier manchon de raccordement (29, 29b, 29f).

9. Système de vannes médical (1a à 1d, 1f) selon l'une quelconque des revendications précédentes, **caractérisé par** un deuxième manchon de raccordement (32, 32b à 32d, 32f) avec un alésage longitudinal (33, 33b à 33d, 33f) destiné à recevoir de façon étanche aux fluides une évacuation de vanne (34), l'alésage longitudinal (33, 33b à 33d, 33f) étant relié en écoulement de fluide au passage (11) et le deuxième manchon de raccordement (32, 32b à 32d, 32f) étant fixé de telle sorte sur le côté avant et dans le plan coaxial par rapport à l'axe de rotation au niveau du corps de vanne (3a à 3d, 3f) que le corps de vanne (3a à 3d, 3f) puisse tourner par rapport au deuxième manchon de raccordement (32, 32b à 32d, 32f).

10. Système de vannes médical (1a) selon la revendication 8 et/ou 9, **caractérisé en ce que** le premier manchon de raccordement (29) et/ou le deuxième manchon de raccordement (32) est fixé/sont fixés fixement en rotation au niveau du carter de vanne (3a), le premier et/ou le deuxième manchon de raccordement (29, 32) comportant respectivement une plaque de recouvrement (36, 36) et étant disposé/disposés de telle sorte que respectivement une fente radiale (37, 38) réalisée entre le corps de vanne (3a) et le carter de vanne (2a) est recouverte sur le côté avant.

11. Système de vannes médical (1d) selon la revendication 9, **caractérisé en ce que** le corps de vanne (3d) comporte un canal de liaison (41) relié en écoulement de fluide au canal de rinçage (Kd) dans au moins une des positions de médicament et que le deuxième manchon de raccordement (32d) comporte un alésage transversal (42) passant de la surface extérieure périphérique du manchon de raccordement (32d) jusque dans son alésage longitudinal (33d), le deuxième manchon de raccordement (32d) pouvant tourner depuis la position de médicament respective du corps de vanne (3d) par rapport à celui-ci et pouvant être relié au canal de liaison (41) dans une position de rétro-amorçage dans laquelle l'alésage transversal (42) est relié en écoulement de fluide.

12. Système de vannes médical (1f) selon l'une quelconque des revendications précédentes, **caractérisé par** la présence d'un élément d'étanchéité (43) disposé entre la surface périphérique extérieure de corps (7) et la surface périphérique intérieure de carter (4) de façon à étanchéifier un interstice radial prévu entre le corps de vanne (3f) et le carter de vanne (2f).

13. Système de vannes médical (1e) selon l'une quelconque des revendications précédentes, **caractérisé par** la présence d'un dispositif de recouvrement (44) disposé sur le côté avant au niveau du carter de vanne (2e) avec un corps de base (45) doté d'une ouverture traversante (46) destinée à recevoir une conduite flexible ainsi qu'avec au moins un élément de recouvrement (47) disposé dans la région d'au moins une des admissions de médicament (E1e à E4e), agencé de telle sorte qu'il limite l'accessibilité manuelle à l'admission de médicaments (E1e à E4e), l'élément de recouvrement (47) comportant notamment un moyen de liaison (50) agencé de telle sorte qu'une conduite d'amenée de médicament puisse être arrêtée par complémentarité de formes au niveau de l'élément de recouvrement (47) et/ou l'élément de recouvrement (47), pouvant tourner, à l'aide d'un moyen de fixation (49) par rapport au corps de base (45) fixé à lui, notamment à l'aide d'une charnière à film (49).
